Europäisches Patentamt

European Patent Office

Office européen des brevets

(19)

(11) Publication number: **0 405 284 A2**

# EUROPEAN PATENT APPLICATION

(12)

(21) Application number: **90111468.6**

(22) Date of filing: **18.06.90**

(51) Int. Cl.5: **A61L 29/00**

(30) Priority: **29.06.89 US 372775**

(43) Date of publication of application:
**02.01.91 Bulletin 91/01**

(84) Designated Contracting States:
**DE FR GB**

(71) Applicant: **HERCULES INCORPORATED**
**Hercules Plaza**
**Wilmington Delaware 19894(US)**

(72) Inventor: **Greiner, Richard W.**
**48 Clifton Drive**
**Kennett Square, Pennsylvania 19348(US)**

(74) Representative: **von Füner, Alexander, Dr. et al**
**Patentanwälte v. Füner, Ebbinghaus, Finck**
**Mariahilfplatz 2 & 3; 3**
**D-8000 München 90(DE)**

(54) Pharmaceutically impregnated catheters.

(57) The present invention relates to a catheterization system which incorporates impregnating a pharmaceutical into a catheter by contacting the catheter with a volatile solvent or swelling agent at or near supercritical conditions of the swelling agent with a mixture of the pharmaceutical and the swelling agent. The catheter is capable of providing prolonged protection of localized tissue areas.

EP 0 405 284 A2

## PHARMACEUTICALLY IMPREGNATED CATHETERS

The present invention relates to catheters that are made of a polymer material which have been impregnated with a pharmaceutical. In particular, the present invention relates to a catheterization system which incorporates impregnating a pharmaceutical into a catheter by contacting the catheter with a volatile solvent or swelling agent. The catheter is capable of providing prolonged protection of localized tissue areas.

Catheters are tubular, flexible, open-ended, medical instruments which are used for shunting fluids into and out of the body of an animal. Such catheters may have preformed ends to facilitate selective locating, as in a renal or coronary vessel, from a remote entry site, and may be named according to the site of entry and destination.

A problem that is known to exist in catheterization is that the insertion of a catheter may lead to acute microbial infection which would require treatment by an antibiotic. Much attention has been given as how to minimize this problem.

Another area of concern in the use of a catheter, is the occurrence of tissue irritation at the site of insertion. This irritation can be of a short term effect such as pain or discomfort associated with friction generated by the insertion of the catheter, or it can be of a long term nature such as the effects associated with a lesion or sore formed at the point of contact between the catheter and the local tissue area. Such lesions or sores may arise when pressure is applied over an extended time. Pressure results in shear and compressive forces on tissues, blood vessels, and the lymphatic system. When blood vessels close as a result of these forces, flow of blood stops and tissues become starved of nutrients, particularly oxygen. Eventually tissues die and the affected area becomes sensitive to microbial infection. Such an occurrence is common in tissue areas that have been in prolonged contact with a catheter.

Known methods of applying pharmaceuticals to catheters include osmotic swelling, coating the surface of a polymer with an antibiotic and a chemical binder, applying a film or solid particles which includes an antithrombotic, applying a coating capable of adsorbing a microbiocide, incorporating microporous walls into the catheter for holding a medicament, and treating the surface of the medical device with a compound having reactive functional groups followed by a second coating of a hydrophilic polymer.

According to the present invention there is a method of impregnating a catheter, made of a polymeric material, with a pharmaceutical comprising the steps of immersing the catheter into a saturated solution of a pharmaceutical in a pharmaceutically acceptable solvent, contacting the catheter at or near supercritical pressure and temperature conditions of the solvent with the saturated solution, and reducing the pressure from the supercritical pressure condition to release the solvent from the catheter, thereby leaving the pharmaceutical impregnated into the catheter. The presence in the catheter of impregnated pharmaceuticals, such as antibiotics, reduces problems encountered in the use, particularly extended use, of catheters in animals. For example, a catheter impregnated with an antibiotic in accordance with the present invention inhibits microbiological infection and possible damage in the tissue contacting the catheter.

In addition to minimizing bacterial infection, a catheter impregnated in accordance with the present invention reduces localized pain and inflammation and retards tissue necrosis which may be associated with prolonged insertion of the catheter into the body.

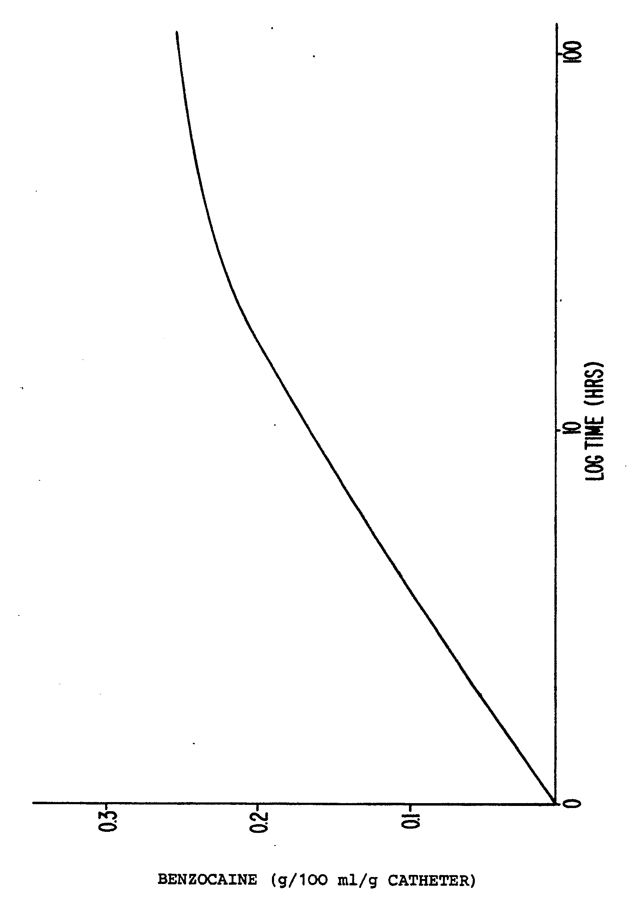

These and other features of the present invention will be readily apparent to those having ordinary skill in the art from the following detailed description of the preferred methods and embodiments of the present invention taken in conjunction with the accompanying drawing. Fig. 1 is a graph which illustrates the results of the rate of release of benzocaine from various polymer compositions used in the manufacture of the catheter. Details of Fig. 1 are included in the examples.

The impregnated polymer is a catheter that is to be inserted into the body of an animal for shunting fluids into and out of body cavities such as the alimentary canal, cerebrospinal cavities, urinary tract, Eustacian tube, reproductive tracts, cranial cavity, ductal tracts, endocrine system, lymphatic system, respiratory system, cardiovascular system, skeletomuscular system, and integumentary system. The catheter can have various forms and structures, which will be apparent to the skilled artisan. Examples of these types of catheters are urinary catheters and percutaneous lead devices such as a shunt, cannulae or wire. An antibiotic or an anti-inflammatory agent is preferably impregnated into the urinary catheter. An antibiotic, anti-inflammatory agent or an anesthetic is preferably impregnated into the percutaneous lead device. The percutaneous lead device can then be implanted through the skin for providing access to veins and arteries and to administer drugs and fluids.

The catheter of the present invention is made of a polymer composition. Such polymer compositions include polypropylene, polyurethane, polytetrafluoroethylene and silicone rubbers.

The useful polymeric catheter materials are preferably impregnated with the pharmaceutical by immersing the catheter in a saturated solution containing the pharmaceutical dissolved in a highly volatile, pharmaceutically acceptable solvent capable of swelling the polymer materials or more preferably by using a pharmaceutically acceptable swelling agent as a deposition medium as taught in U.S. Pat. No. 4,958,006.

A saturated solution is the most concentrated solution that can remain in the presence of an excess of the dissolved solvent. Highly volatile solvents are considered to be those materials which have high vapor pressures at about room temperature. Examples of preferred solvents include, but are not limited to carbon dioxide, nitrous oxide, nitrogen, ethylene, pentane, hexane, cyclohexane, methanol, ethanol, diethyl ether, methyl chloride, methylene chloride, water, isopropanol.

Contacting the catheter at or near supercritical conditions of the swelling agent with a mixture of the pharmaceutical and the solvent or swelling agent permits rapid impregnation of the mixture into the catheter. The greater the swelling, the quicker and more complete the diffusion of the mixture. The efficiency of the swelling is increased with increasing temperature at a constant pressure. The range of temperatures is purely practical and is defined by the properties of the supercritical solvent being used, the effect of temperature on the solubility of the pharmaceutical agent in the supercritical fluid and the effect of temperature on swelling of the polymer by the supercritical fluid. ·The optimal temperature will be determinable by those of ordinary skill in the art. It is preferred that the catheter be contacted by the mixture of the pharmaceutical and swelling agent at a temperature range of from about 20° C below the critical temperature of the swelling agent up to about 100° C. (Critical temperature is defined as the temperature above which a substance has no liquid-vapor transition.)

However, a temperature of at least 40° C will be employed in order to ensure a convenient rate of swelling, with an upper limit of about 60° C being preferred.

Pressure is also determined by practical considerations such as the properties of the particular solvent used and by the cost and availability of equipment. The optimal pressure will be determinable by those of ordinary skill in the art. It is preferred that contacting the catheter with the mixture of the pharmaceutical and the swelling agent be carried out in a range of about 35.2 $kg/cm^2$ to about 703 $kg/cm^2$, with about 70.4 $kg/cm^2$ to about 35.6 $k./cm^2$ being preferred.

After contacting, the volatile swelling agent is separated from the catheter, leaving the pharmaceutical behind. Because of the volatility of the swelling agents employed, separation is easily accomplished by lowering the pressure.

A pharmaceutical that is dissolvable in a pharmaceutically acceptable, swelling solvent or highly volatile, pharmaceutically acceptable swelling agent is useful in accordance with the present invention. Non-limiting examples include antibiotics, anti-inflammatory agents, analgesics, anti-fungal agents, topical anesthetics, antiseptics, vasodilators, antispasmodic agents, and growth factors such as methenamine, nalidixic acid, cinoxacin, norfloxacin, pifloxacin, gentamicin sulfate, clindamycin phosphate, benzocaine, triethanolamine salicylate, hydrocortisone, trolamine salicylate, theophylline, dipyridamole, minoxidil, nylidrin hydrochloride, nifedipine, erythrityl nitrate, nitroglycerine, cinepazide, isoxsurpine, trolnitrate phosphate, isosorbide dinitrate, betahistine, pentaerythritol tetranitrate, epidermal growth factor (EGF), transforming growth factors alpha and beta (TGF- alpha and -beta), fibroblast growth factors alpha and beta (FGF-alpha and -beta), cartilage inducing growth factors A and B (CIF-A and -B), tumor angiogenesis factor (TAF), and platelet derived wound healing factors (PDWHF).

The amount of pharmaceutical impregnated into the polymer depends upon various factors such as length of time desired for administration and the minimum and maximum effective dose. The amount of pharmaceutical varies between about 1 and 50% by weight of the catheter. Preferred ranges depend explicitly on the pharmaceutical agent, but generally will be about 5 to 15% by weight of the catheter.

The pharmaceutical impregnated into the catheter is capable of being released from the catheter at a prolonged and controlled rate. Preferably, the pharmaceutical is released at a rate of about 0.01 to 30.0 wt % of the agent per hour based on total weight of the catheter plus impregnated material.

Preferably, the polymer is preformed into the desired catheter end product before impregnation with the pharmaceutical. This avoids any loss of pharmaceutical that would have occurred during manufacture of the desired article. Commercially available, preformed catheters that can be impregnated with a pharmaceutical in accordance with the present invention include Bardex™ latex Foley catheters with silicone coating (made by C. R. Bard, Inc.); Bardex™ latex Foley catheter with teflon coating (made by C. R. Bard, Inc.); all silicone Foley catheters; long-term, indwelling catheters; balloon-type cardiovascular catheters; coronary profusion catheters; hemodialysis catheters;

and angiographic catheters.

The catheter of the present invention can be conveniently contained in a sterilized kit for dispensing. Preferably, the kit is a sterilized wrapper that is impermeable to air, moisture, and bacteria. Examples of useful wrappers are Tyvek™, polyethylene or polyvinylidene chloride pouches and polyolefin trays.

The following non-limiting examples are provided to more clearly describe the present invention. In the examples, all part and percentages are by weight unless otherwise indicated.

EXAMPLE 1

A commercially available polyurethane catheter is mounted in a stainless steel cage, placed in a reactor, and 1 g of benzocaine is added to the reactor. The reactor is closed, heated to 40°C, and pressurized with carbon dioxide to 1200 psig. After the contents of the reactor are agitated at 1,000 rpm for 4 hours, the reactor is cooled and depressurized.

The catheter is found to contain 25% benzocaine by weight, as determined by weight gain. The presence of benzocaine is confirmed by extracting the benzocaine with a pharmaceutically acceptable solvent and analyzing the extracted solution by UV analysis.

The rate of release of benzocaine from the catheter is measured by the following procedure. The catheter is placed in a beaker containing 100 ml of water, and the contents of the beaker are slightly agitated with a magnetic stirrer. At given times, an aliquot is removed, placed in a cuvette and the UV absorbance @ 285 nm is measured. The concentration is determined by comparison with a standard calibration curve. The results are summarized in Fig. 1. These results demonstrate the prolonged release rate characteristics of the impregnated catheter.

EXAMPLE 2

The process described in Example 1 is repeated except that benzene is replaced with a slurry solution of ethanol and 1 gram of benzene.

The catheter is analyzed for weight gain and is found to contain 42% benzocaine.

EXAMPLE 3

A Hickman right atrial catheter (made by Evermed of Medina, WA) is impregnated according to the process described in Example 1. This catheter is made of Silastic™, a silicone rubber compound, which is a product of Dow Corning.

EXAMPLE 4

A latex rubber catheter (made by C. R. Bard, Inc.) is impregnated with gentamicin according to the process described in Example 1.

EXAMPLE 5

A commercially available polyurethane catheter is impregnated with Cyclospasmol™ (a cyclandelate vasodilator made by Wyeth Laboratories) according to the process described in Example 1.

EXAMPLE 6

A commercially available polyurethane catheter is impregnated with 1.0 g sulfathiazole, an antibacterial agent, according to the process described in Example 1.

EXAMPLE 7

A commercially available polyurethane catheter is impregnated with Fluocinonide™ (an anti-inflammatory agent made by Syntex Labs) according to the process described in Example 1.

**Claims**

1. A method of impregnating a catheter, made of a polymeric material, with a pharmaceutical comprising the steps of:

(a) immersing the catheter into a saturated solution of a pharmaceutical in a pharmaceutically acceptable solvent;
(b) contacting the catheter at or near supercritical pressure and temperature conditions of the solvent with the saturated solution; and
(c) reducing the pressure from the supercritical pressure condition to release the solvent from the catheter, thereby leaving the pharmaceutical impregnated into the catheter.

2. The method of claim 1, wherein the catheter is made of silicone rubber, polyethylene, polytetrafluoroethylene or polyester.

3. The method of claim 1, wherein the pharmaceutical is selected from the group consisting of an antibiotic, vasodilator, anti-inflammatory agent, growth factor, analgesic, antispasmodic, and a mixture thereof.

4. The method of claim 1, wherein the amount of pharmaceutical is impregnated into the catheter in a range of about 1 to 50 wt %.

5. The method of claim 1, wherein the catheter is contacted in a temperature range of about 20°C below the critical temperature of the solvent to about 60°C.

6. The method of claim 1, wherein the impregnation takes place in a pressure range of 70.4 to 351.6 kg/cm².

7. A pharmaceutically impregnated catheter produced by the process of any of the preceeding claims.

8. A method of shunting bodily fluids of an animal through the pharmaceutically impregnated catheter of claim 7.

9. The method of claim 8, wherein the fluids are shunted through a body cavity of 10the urinary tract.

10. The method of claim 8, wherein the fluids are shunted through a body cavity of the respiratory system.